(19) Europäisches Patentamt
European Patent Office
Office européen des brevets

(11) EP 0 420 232 B1

(12) EUROPEAN PATENT SPECIFICATION

(45) Date of publication and mention of the grant of the patent:
**26.03.1997 Bulletin 1997/13**

(51) Int Cl.6: **C07H 17/075**, A61K 31/70

(21) Application number: **90118552.0**

(22) Date of filing: **27.09.1990**

(54) **Water soluble derivatives of glycoside bioflavonoids, processes for their preparation and related pharmaceutical compositions**

Wasserlösliche Derivate der Bioflavonoidglykoside, Verfahren zu ihrer Herstellung und pharmazeutische Präparate davon

Dérivés du glycoside de bioflavonoides, solubles à l'eau, procédé pour leur préparation et compositions pharmaceutiques les renfermant

(84) Designated Contracting States:
**AT BE CH DE DK ES FR GB GR IT LI LU NL SE**

(30) Priority: **28.09.1989 IT 2186789**

(43) Date of publication of application:
**03.04.1991 Bulletin 1991/14**

(73) Proprietor: **EUROPEAN PHARMACY Srl**
**20100 Milano (IT)**

(72) Inventors:
- **Scapini, Giancarlo**
  **I-40123 Bologna (IT)**
- **Tumiatti, Vincenzo**
  **I-37020 Cerro Veronese (VR) (IT)**
- **Ghedini, Nadia**
  **I-40133 Bologna (IT)**
- **Andrisano, Vincenza**
  **I-40139 Bologna (IT)**

(74) Representative: **Dragotti, Gianfranco et al**
**SAIC BREVETTI s.r.l.**
**Galleria San Babila, 4/D**
**20122 Milano (IT)**

(56) References cited:
**EP-A- 0 121 489** **GB-A- 1 179 019**

- **CHEMICAL ABSTRACTS, vol. 70, no. 16, 21st April 1969, page 398, abstract no. 78325z, Columbus, Ohio, US, P.W. THIES et al.**
- **CHEMICAL ABSTRACTS, vol. 69, 1968, page 10042, abstract no. 107018p, Columbus, Ohio, US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to water soluble derivatives of glycoside bioflavonoids, particularly of the rutinosides of 3,3', 4', 5,7-pentahydroxyflavone and of 5,7 , 3'-trihydroxy -4'-methoxyflavone.

Some bioflavonoids of glycoside nature (Q) have found widespread pharmaceutical use for the treatment of pathological conditions characterized by an anomalous increase of the capillary fragility (P vitamin activity). Among them, the flavonic rutinosides, rutin and diosmine, are the commercial compounds of utmost interest as regards the use in pharmaceutical and cosmetic field;

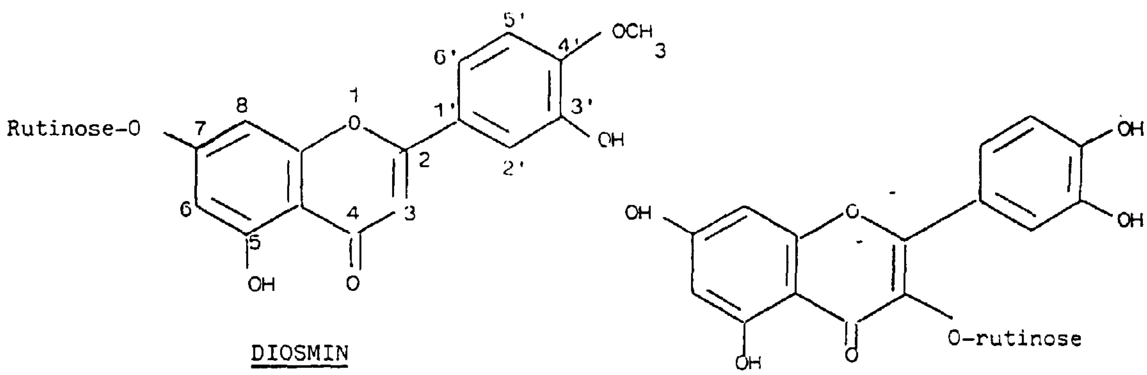

DIOSMIN

RUTIN

Where in rutinose is

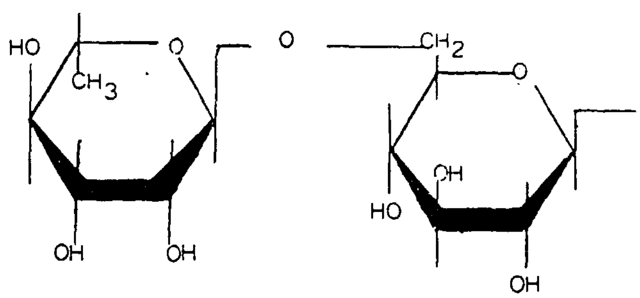

For them what has been to date proposed can be resumed as follows:

- rutin, (3-rutinoside of 3, 3', 4', 5, 7-penthahydroxyflavone), is therapeutically used for its property of reducing the fragility of capillary vessels. Extended treatments of patients the capillary fragility of whom was evidenced by cutanenous, pulmonary and gastrointestinal haemorrhagies have led to satisfactory results. The indications thereof as a drug can be resumed as follows: all the syndromes induced from increased fragility and permeability of capillary vessels and, particularly haemorrhagic occurrence in patients affected by hypertension; in the patients being subjected to therapy with arsenical substances, salicylates and X rays; diabetic retinitis; haemorrhagic teleangiectasis, pulmonary haemorrhagies of uncertain origin; anti-haemorrhagic prophylaxis before surgical interventions.
- diosmin, (7-ramnoglycoside of 5,7,3'-trihydroxy- 4'-methoxyflavone), of semisynthetic origin, has like therapeutical uses in the phlebitis and as coadjuvant agent in the therapy of the states of capillary fragility (varices, phlebitic complications, internal and external haemorrhoids , ecchymosis, haematoma, purpura, etc.).

Both substances, moreover, can be considered as devoid of toxicity.

As most of the heteroside bioflavonoids , rutin and diosmin are poorly soluble or pratically insoluble in the most common organic solvents (alcohol, acetone, ethyl acetate, ether, chloroform , carbon sulfide , benzene, etc.), very poorly soluble in water at room temperature , soluble in hot methanol, pyridine , dimethylformamide and alkaline solutions.

These properties of hydro- and lipo-insolubility limited their absorption "in vivo" both by topical and by systemic route. In this sense, in order to improve their bioavailability after oral administration, some derivatives have been patented involving the phenolic hydroxyls of the flavonic residue (phenolic ethers) and some of these derivatives are presently therapeutically interesting.

The object of the present invention is to provide biodegradable derivatives involving the glycoside residue of the heteroside , in order to improve the partition profile of the same heterosides, promoting the absorption and the carrying through the several physiological barriers and maintaining unchanged, or readily restorable "in vivo", the flavonic residue of the heteroside , which is considered as responsible of the biological activity.

A first object of the present invention are thus the derivatives of glycoside bioflavonoids having general formula

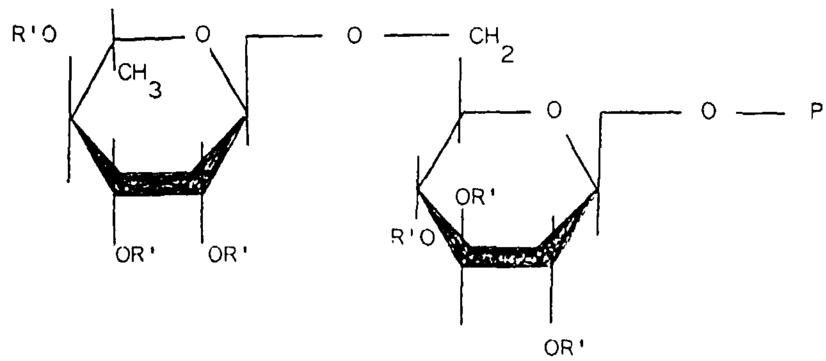

I

wherein P represents the flavonic residue containing phenolic hydroxyls and R' represents H, (CO-R-CO-O-X), in which X is selected among H, the cation of a pharmaceutically acceptable base and the radicals of polyoxyethylenglycols, polyoxypropylenglycols, and their monomethyl ethers, and R is an alkyl or aryl radical.

The hemiesterification of the hydroxyls of the structure being considered with aliphatic or aromatic dicarboxylic acids makes it as a matter of fact possible to obtain derivatives in which free carboxylic functional groups are present.

The number of the ester-carboxylic residues which are introduced in the molecule of the glycoside may vary depending on the reaction conditions and on the stoichiometrical ratios between the reactants, even though it cannot be higher than the number of hydroxy group of the molecule itself (for example in the case of the rutin it varies between 1 and 10). Generally, however, the products which are obtained represent a mixture of compounds having several degrees of functionalization, to which an average molecular weight (PM) corresponds which can be determined by osmometry in a suitable solvent. Under the most convenient reaction conditions (see examples) the esterification involves the aliphatic hydroxyls of the glycidic residue, as demonstrated by the fact that in the thus obtained derivatives the reaction of the phenols with ferric chloride remains positive. Also the U.V. spectrum of the compounds maintains the properties of the chromophore group of the bioflavonoid Q; this is useful in view of the pharmacological answer, the flavonic residue being considered has responsible of the interaction with the receptors and consequently of the biological activity. As a matter of fact, thus, the functionalizations proposed in the present invention are mainly acting onto the bioavailability of the bioflavonoids, without interference with the pharmacological answer.

The presence of the free carboxyl in the thus introduced ester residues, permits from one side hydrosoluble salt (for example sodium salts) of the novel derivatives which are disclosed to be obtained, and from the other side to chemically attach to the same carboxyls suitable "carriers" which in that case, have been found among polyoxyethylen glycols, polyoxypropylene glycols or their O-monomethylethers which are notoriously devoid of systemic and topical toxicity.

With the derivatives esterified with the above-mentioned carriers, in which the dicarboxylic acid has the function of asymmetrical bis-ester bridge between the hydroxyls of the active substance and the end hydroxyl of the carrier itself, particular advantages of bioavailability are obtained , apart from those of industrial processing.

As a matter of fact:

- the obtained product is at the same time water soluble and liposoluble;
- the esterified polyether has the function of "carrier" in the processes of absorption through the several physiological barriers , including the skin;
- "in vivo", the asymmetrical bis-ester bond of the dicarboxylic acid with a hydroxyl of the active principle from one side and with the end hydroxyl of one of the carriers as defined above on the other side, is enzimatically cut, whereby the original drug is slowly and gradually restored ("retard" effect). The carrier by making it easier the carrying of the active principle through most of the physiological barriers permits parts of the organism to be reached which are normally not accessible for the bioflavonoids.

In turn the process for the preparation of the derivatives according to the present invention involves the following steps:

a) reaction of the glycoside with the anhydride of an aliphatic or aromatic dicarboxylic acid in the presence of an organic base, under controlled conditions of temperature and reaction time, taking care that the reaction environnment is anhydrous and whatever photochemical action on the starting glycoside is prevented;
b) reaction of the free carboxyls of the resulting ester-carboxylic derivatives with a reactant selected among the pharmaceutically acceptable bases, polyoxyethylenglycols, polyoxypropylenglycols and their monomethylethers.

In the case of reaction with a pharmaceutically acceptable base, the conversion of the carboxylic derivatives into the corresponding water soluble salts can be carried out in water solution which , in the cases of an inorganic base, is selected among bicarbonate , carbonate and hydrate (for example and preferably of sodium), followed by an evaporation of the solvent or addition , after concentration, of acetone or alcohols. In the case of an organic base, the reaction takes place by direct addition of the latter in a suitable solvent.

The esterification of the free carboxyls of the ester-carboxylic derivatives with polyethers, carriers as defined above does not lead to favourable results if most of the commonly known methods for the esterification are followed; good results are obtained , on the contrary, by using the active amides of the carboxylic derivative (imidazolide , benzotriazolide, etc.) acting as exchange agents in the esterification processes. Scheme 1 illustrates the synthetic routes which to date resulted to be more suitable:

- for the hemiesterification of the alcoholic hydroxyls of glycidic bioflavonoids (Q) or for the mono-hemiesterification of the end alcoholic hydroxyls of polyoxyethylenglycols and polyoxypropylenglycols or of their mono-0-methylethers of commerce (reactions A and B);
- for the conversion of polyhemiesters of the glycidic bioflavonoids into the corresponding water soluble salt (second step of the reaction A)
- for the conversion of the hemiesters of PEG into the corresponding anhydrides (second step of the reaction B);
- for the conversion of the polyhemiesters of the glycidic bioflavonoids into the corresponding asymmetrical bis-esters with PEG through the intermediate formation of the corresponding active amide (in this case imidazolide) (reaction C);
- for the preparation of the same asymmetrical bis-esters of dicarboxylic acids with carriers as defined above and with alcoholic hydroxylis of glycidic bioflavonoids through the forming of the active amide (in this case imidazolide) of the hemiester of above-mentioned carriers (reaction D) or through the reaction of the bioflavonoid Q with the anhydride of the carrier hemiester (reaction E) with recovery of one mole of carrier hemiester.

SCHEME 1

REACTION A

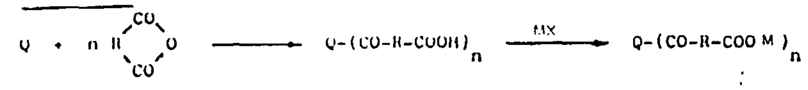


REACTION B

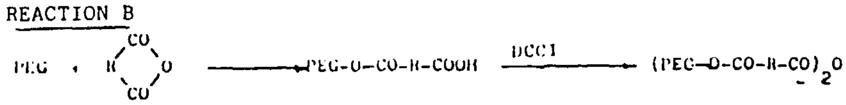


REACTION C

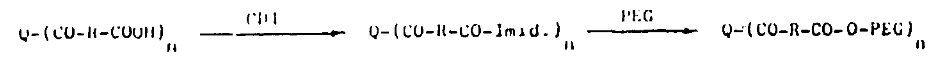


REACTION D

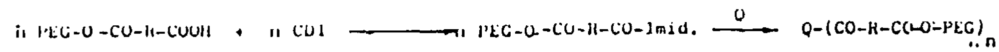


REACTION E

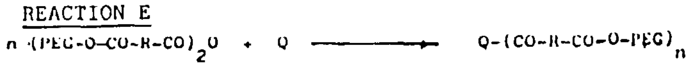

PEG= polyoxyethylenglycols or polyoxypropylenglycols and their mono-O-methylesters;
Q= rutin (R), diosmin (D) or other glycosidic bioflavonoid
R= alkyl or aryl biradical of the anhydride of the dicarboxlyic acid
DCCI= dicyclohexylcarbodiimide or corresponding compounds with dehydrating action
CDI= carbonyl diimidazole or related compounds adapted to form active amides;
M= cation of a pharmacologically acceptable salt or radical or an organic base and
X= OH, an anion of a carboxylic acid adapted for the reaction of ionic exchange in a suitable organic solvent or H.

The above reactions are more detailedly illustrated hereinafter.

REACTION A

Exhausting acylation of glycidic bioflavonoid (Q) with succinic (S) or glutaric (G) anhydride.

In a flask with emery or frosted neck, having refluxing cooler and protected from the light , a suitable amount of etheroside Q (0.05 mole) is dissolved under magnetic stirring in dimethylformamide made anhydrous on $CaSO_4$ (DMF; 200 ml) and treated with a 10 times molar excess (0.5 mole) of internal anhydride of the selected dicarboxylic acid (G or S) and at with a 10.5 molar excess (0.525 moles) of pyridine made anhydrous on $CaSO_4$ (Py). The reaction flask , provided with the refluxing cooler and isolated from the external moisture by means of a closure with a small Sikkon® pipe is immersed in a silicone bath heated to 55-60°C.

The reaction mixture is kept under constant stirring at such temperature for 36-48 hours, and left to spontaneously cool down to room temperature during the last 4-6 hours. The cool solution is pouered, slowly and under constant stirring , in a becker containing ground ice and made acidic with concentraded HCl (1800 ml of ice + 200 ml of 37% HCl; final solution about 1N). A tar like gold-yellow product is separated, which is washed by macerating and decanting with portions of distilled water (3 x 300 ml) and then treated with water (300 ml) and moreover, in portions and under stirring, with sodium bicarbonate (30 g; 0.35 mole) until the gas development ceases and the complete dissolution takes place. The resulting solution is degassed by stirring for 30 minutes at room temperature, then filtered and cautiously made acidic,( owing to bubbling and foaming) with 37% HCl (35 ml, 0.35 moles); at such a pH (less than 1) the tar-like product precipitates again, and is again washed with portions of distilled water (3x 200 ml), then dissolved in methanol (200 ml) and made anhydrous on $Na_2SO_4$. The methanolic solution is filtered in a flask with frosted neck and dried under vacuum (Roctavapor) at a bath temperature of 55°C± 3°C. The product is then subjected to a number of anidryfying steps by dissolution is acetone and evaporation to dryness under vacuum at the indicated temperature (3x

150 ml of acetone) and then is digested in hot condition (55°C) under petroleum ether and the solvent is removed by decantation (2-3 times with 150 ml of petroleum ether), until it takes a semicrystalline aspect by removal of the solvent in a Roctavapor (T ≤60°C ) and maceration. The drying at the same temperature under high vacuum (0.13 millibar =0.1 mm/Hg) up to constant weight , gives a yellow, semicrystalline and low melting product. Yields: 70-85% of the theoretical value.

The product is characterized both qualitatively and semiquantitatively by the spectrum of nuclear magnetic resonance in DMSO-d $_6$ by the positive reaction with $FeCl_3$ (free phenolic OH) and the by pattern of the spectro photometric curves in the area of the UV and of the visible light which confirm, in a solution of 95% ethanol, the presence of the same chromophore group present in the bioflavonoid Q.

The comparison of the UV spectra in 0.1 NaOH of the adduct of the present invention and of the bioflavonoid shows in both cases the same spectrum modifications in the spectrum area of 280-400 nm, which are attributed to the alkaline opening of the flavonic heterocycle (forming of calconic derivatives): it indicates that during derivatization process, no alteration of the molecular residue which is responsible of the chromophoric and pharmacophoric activity took place.

The quantitative characterisation of the reaction adducts is provided from the titration of the free carboxylic functional groups (ethanolic solution; titrating agent 0.1 NaOH; indicator phenolftalein), from the spectrophotometric dosage at the absorption peaks at the UV and visible range, both in 95° ethanol solution and in 0.1 NaOH solution, up 30 minutes, and by the determination of the average weight ($\overline{PM}$) by osmometry.

Water soluble sodium salt of the glutaric and succinic polyhemiesters.

A suitable amount of above described polycarboxylic derivatives,(0.02 moles) is dissolved in a flask in absolute ethanol (180ml) and added, under vigorous stirring at room temperature and by means of drop adding funnel, with the stoichiometrical equivalent of NaOH (tablets) dissolved in water (20 ml).

By keeping constant the stirring and still by means of the drop addition funnel, a suitable amount of acetone ( 400 ml) is slowly added (20 minutes). The sodium salt is separated , which is filtered on Buchner, washed with acetone /absolute ethanol 2/1, dried at 90°C in a thermostatic device under forced air circulation for 8 hours and ground in a mortar. The resulting products are yellow, of powdery aspect, highly soluble in water and practically not soluble in most of the common organic solvents. The yield is practically the theoretical one. Alternatively the salification can be carried out by reaction of cationic exchange with the sodium salt of aliphatic acid soluble in anhydrous organic solvent (for example sodium hexanoate). As an indicating example , the sodium salt D-S-COONa (see table 1; compound 10) can be also obtained by dissolving 7 (0.002 moles) in methanol (30 ml) and treating at 50°C with a solution in the same solvent (30ml) of sodium caproate excess (0.05 moles). After stirring in hot conditions for about 20 minutes, the mixture is dried under vacuum (Roctavapor) and taken with absolute ethanol (50 ml), the mixture being boiled under stirring and refluxed for 15 minutes. Through the filtration on Buchner in hot condition and washing with portions of hot absolute ethanol (3 x15 ml) the product is obtained with high purity degree. The salification yields are higher than 95% of theoretical value.

The ethanolic filtrates, evaporated to dryness under vacuum, taken wich N HCl and extracted with ethyl acetate, permit , through the evaporation of extraction solvent, the practically thereotical recovery of the caproic acid.

The structure of the sodium salt of the described glutaric and/or succinic polyhemiesters, is demonstrated from the study of the nuclear magnetic resonance spectra in $D_2O$; by the positive reaction with $FeCl_3$ : by the centesimal analysis of C and H and by the spectrophotometrical analysis with Na flame; by the pattern of the UV spectrum in 0.1 N NaOH solution and by the spectrophotometric dosage in the UV and visible range of the percentage of active sostance Q.

REACTION B

Glutaric and succinic hemiesters of polyoxyethylenglycol 350-O-methylether carriers.

In a 2000 ml flask with emery plug, 0.2 moles of a carrier as defined above are dissolved in 800 ml of chloroform devoid of EtOH and made anhydrous on $CaH_2$ and treated with 0.4 moles of glutaric anhydride (G) or succinic anhydride (S) and with 0.4 moles of pyridine made anhydrous or $Na_2SO_4$ (Py). After addition of same glass beads, the reaction mixture is refluxed for 24 hours, taking care that the temperature of the external path is not higher than 60°C. Then the solvent is recovered by evaporation under vacuum to dryness (temperature of the external bath: 65°C±3°C) and the residue is taken with 600 ml of dionized water. The aqueous solution is added, at room temperature and under costant stirring, with 42.2 g (0.4 moles) of sodium carbonate and, after stirring for 30 minutes and cooling to 5°C , 100 ml of 37% HCl (1.0 moles). The acidic solution is extracted with 3 x 150 ml of chloroform. The combined chloroform extracts are washed with 3 x 200 ml of water and made anhydrous on $Na_2SO_4$. By removing the solvent under vaccum and oleous, colorless or slightly yellow residue is obtained, which, upon being washed in hot condition by decantation

with petroleum ether or ligroin (3 x 100 ml) and brought to dryness under high vacuum (0.1 mm/Hg) up to constant weight, corresponding to the desired hemiester (nuclear magnetic resonance spectrometry in CDCl ). The yields vary in the range of 92-96% of the theoretical value.

The determination of the average molecular weight ($\overline{PM}$) per osmometry and titration in ethanol/water/1/1 with 1 NaOH, with phenolftalein as indicator , show the optimum degree of purity of hemiesters (purity: 99.2-100.5%).

Anhydrides of glutaric and succinic hemiesters with carriers

0.084 moles of hemiester 1 or 3 are dissolved in 160 ml of acetone made anhydrous on CaSO and treated, under stirring, with 8.2 (0.004 moles) of dicyclohexyl carbodiimide (DCCI) dissolved in 40 ml of anhydrous acetone. Upon isolating the reaction flask from the external moisture by means of Sikkon® valve, the mixture is maintained under stirring at room temperature for 12 hours, then it is heated up to beginning boiling (about 55°C) for further 24 hours, leaving the mixture to cool down to room temperature during the last 4 to 5 hours. The separated dicyclohexylurea is removed by filtration on Buchner , washed with acetone and dried in thermostatic device at 80°C ; the weight thereof indicates in semiquantitative manner the degree of completion of the reaction (under the indicated experimental conditions 92-96% of the hemiesters is converted into the corresponding anhydride). The acetonic filtrate is brought to dryness under vacuum at 60°C (recovery of the solvent) and the residue is washed by decantation under hot condition with 2 x 100 ml of petroleum ether, it being then brought to dryness under high vacuum (0.1 mm/Hg) up to constant weight. The residual colorless or slightly yellow oil is the technical anhydride of the carriers hemiesters, with a sufficient purity degree (92-96%) for the use, without further purification, in the acylation reactions described in scheme 1, the main impurity being the corresponding free acid, which compound does not interfere with the synthesis processes disclosed in the present invention. The study of the nuclear magnetic resonance spectrum of the products and the determination of their $\overline{PM}$ by osmometry confirm their structures and, in semiquantitative manner, their purity degree.

REACTION C

Synthesis of asymmetrical bis-esters Q-[(S or G)-carrier]$_n$ by reaction between glutaric or succinic polyhemisters of heteroside bioflavonoids and polyoxyethylenglycol 350-$\overline{O}$-methylether.

In a flask protected from the light, provided with refluxing cooler and isolated from the external moisture by means of $CaSO_4$ valve, 0.02 mole of polyhemiester obtained from the reaction A are dissolved in dimethylformamide made anhydrous on $CaH_2$ (DMF; 120-200 ml) and treated at room temperature and in portions with 0.12 moles (19.5 g) of carbonyl diimidazole (CDI). Upon completing the addition of amide forming reactant, the solution is maintained under stirring at room temperature until the gas development ceases and it is then degazed (about 30 minutes); then 0.2 mole (70 g) of polyoxyethylenglycol 350-O-methylether made anhydrous on $CaH_2$ or $CaS_4$ are added. The reaction flask is then immersed in silicone bath heated to 55°C ± 3°C and maintained at such temperature under constant stirring, for 24-36 hours, the heating being suspended during the last 4-5 hours. The cool reaction mixture is poered in 800 ml of ground ice, making it acidic by addition of 200 ml of 37% HCl. (Final solution at about 2N). The acidic solution is two times extracted with 200+150 ml of chloroform or methylene chloride , the organic phase is washed with 2 x 100 ml of saturated solution of NaCl and made anhydrous on $CaSO_4$. After filtration in a flask with emery plug the chlorinated solvent is removed, it being recovered under vacuum and at 55°C up to dryness. It results a red-brown oil which is washed three times, by hot stirring and decantation, with 200 ml of $Et_2O$, each time the mixture being brought to dryness under vacuum at 55°C and then likewise, with two portions of 150 ml of petroleum ether. By final treatment under high vacuum (0.1 mm/Hg) at the temperature of 55°C up to costant weight, highly viscous red-brown oils are obtained.

Alternatively, the red-brown oils, reulting from the removal of the halogenated organic solvent , can be purified by dissolution in 300 ml of deonized water, extraction with 3x150 ml of $Et_2O$ of the reaction impurities and/or of the unreacted products, extraction of the bis-esters from the aqueous phase with chloroform or methylene chloride (2x150 ml), washing of the chlorinated organic phase with a saturated solution of NaCl (2x100 ml), anhydrifying thereof on $Na_2SO_4$ removal of the halogenated solvent and washing of the final product with $Et_2O$ and petroleum ether as above described. This second purification method is more effective in the case of reaction adducts which are particularly enriched with hemiesters or anhydrides of carriers as defined above (reaction E).

The yields of bis-ether are 70-85% of the theoretical value. The structure of the obtained asymrrietrical bis-esters is confirmed by the NMR spectra in $CDCl_3$ e/or DMSO-$D_6$ ; by the pattern of the spectrophotometric curves in the UV and visible range in EtOH solution and also through the spectral modification in the range of 280-400 nm in the first 20 minutes after the dissolution of O.1N NaOH; by the positive reaction with a $FeCl_3$ (free phenolic OH) and by the titration in alcoholic environnment of possible free carboxylic residue (titrating agent : 0.1N NaOH; indicator: thymol blue). The determination of the average molecular weight ($\overline{PM}$) and of the content of active principle (Q %) by spectrophotometry at the wave lenght $\lambda_{anal}$ corresponding to the peaks of absorption in the UV and visible range , quantitatively define the structure of the reaction product.

REACTION D

Synthesis of the asymmetrical bis-esters Q-[(S or G)-carrier]$_n$ from the glutaric or succinic hemiesters of polyoxyethylenglycol 350-O-methylether.

By operating as described in the reaction C and using proportional amounts of solvents and reactants , 0.11 mole of a carrier (as defined above hemiester are dissolved in DMF and treated with 0.1 mole of CDI and then with 0.01 mole of glycoside bioflavonoid Q (R or D).

The resulting structures are characterized as described in the reaction C and the determination of the $\overline{PM}$ and of the Q percentage in the mixtures with variable composition of the two compounds confirms that identical structures are obtained, besides the confirmation given by the examination of TLC chromatograms obtained on silica gel plates, using as the eluant acetone/chloroform/acetic acid in the ratio 4.5/4.5/1.

REACTION E

Synthesis of asymmetrical bis-esters Q-[(S or G)-carrier]$_n$ by reaction between the anhydrides of glutaric or succinic hemiesters of polyoxyethylenglycol 350-O-methylether and heteroside bioflavonoids Q.

By operating as described in reaction C, 0.02 moles of anhydride of the carrier (as defined above) hemiesters are dissolved in DMF and treated with 0.02 moles of heteroside bioflavonoid (R or D).

The characterization of the reaction adducts takes place as described in reaction C and the identity of the asymmetrical bis-ester, obtained through the three different synthesis routes , is confirmed from the determination of the $\overline{PM}$ and of Q percentage in mixtures with variable proportion of the three compounds, besides the examination of TLC chromatograms with the experimental method indicated in reaction D.

As non limiting examples (see table 1) the main chemico-physical properties and general synthesis reactions adopted for the preparation of the hemiesters of polyoxyethylenglycol 350-0-methylether with glutaric and succinic acid (compounds 1 and 3) and of the corresponding anhydrides (compounds 2 and 4) ; of glutaric and succinic polyhemiesters obtained through exhaustive acylation of rutin and diosmine (compounds 5, 6 and 7); of their sodium salts (compounds 8, 9 and 10) and lastly of asymmetrical poly-bis-esters of glutaric and succinic acid with polyoxyethylenglycol 350-0-methylether and with rutin or diosime (compounds 11, 12, 13 and 14) obtained by operating according to the three synthesis routes C-D-E indicated in the scheme 1.

TABLE 1

. Main chemical-physical properties of some oligomeric derivatives obtained by reaction between rutin (R), or diosmine (D) and succinic anhydride (S) glutaric anhydride (G) and polyoexyethylen-glycol 350-O-methylether (PEG) (C)

| Compound | No. | THEORETICAL FORMULA | RAW FORMULA | $\overline{PM}$ calc. | $\overline{PM}$ found [a] (dalton) | %Q (U.V.) calc. | %Q (U.V.) found [b] | ASPECT AND SOLUBILITY | Synthesis reaction |
|---|---|---|---|---|---|---|---|---|---|
| PEG-G-COOH | 1 | $MeO-PEG-O-CO-(CH_2)_3-COOH$ | $C_{20}H_{38}O_{11}$ | 454 | 453 | - | - | Colorless viscous oil; soluble in $H_2O$ and common organic solvents | B |
| $(PEG-G-CO)_2O$ | 2 | $/\overline{M}eO-PEG-O-CO-(CH_2)_3-CO/_2O$ | $C_{40}H_{74}O_{21}$ | 890 | 875 | - | - | colorless viscous oil; poorly soluble in $H_2O$ soluble in common organic solvents | B |
| PEG-S-COOH | 3 | $MeO-PEG-O-CO-(CH_2)_2-COOH$ | $C_{19}H_{36}O_{11}$ | 440 | 435 | - | - | colorless viscous oil; soluble in $H_2O$ and common organic solvents | B |
| $(PEG-S-CO)_2O$ | 4 | $/\overline{M}eO-PEG-O-CO-(CH_2)_2-CO/_2O$ | $C_{38}H_{70}O_{21}$ | 862 | 853 | - | - | colorless viscous oil; poorly soluble in $H_2O$ soluble in common organic solvents. | B |
| R-G-COOH | 5 | $R-/\overline{C}O-(CH_2)_3-COOH/_6$ | $C_{52}H_{60}O_{31}$ | 1.180 | 1.179 | 51,65 | 52,8 | semicrystalline yellow-low melting solid, insoluble $H_2O$ soluble in polar and protic organic solvents | A |

TABLE 1 (follows)

| Compound | No. | Theoretical formula | Raw formula | $\overline{PM}$ calc. | found [a] (dalton) | Q% (U.V.) calc. | found [b] | ASPECT AND SOLUBILITY | Synthesis reaction |
|---|---|---|---|---|---|---|---|---|---|
| D-G-COOH | 6 | $D-[CO-(CH_2)_3-COOH]_5$ | $C_{58}H_{63}O_{33}$ | 1.293 | 1.270 | 47,06 | 45,5 | Semicrystalline yellow-low melting solid, insoluble in $H_2O$ soluble in polar and protic organic solvents | A |
| D-S-COOH | 7 | $D-(CO-CH_2-CH_2-COOH)_6$ | $C_{52}H_{57}O_{33}$ | 1.209 | 1.219 | 50,33 | 50,8 | semicrystalline yellow-low melting solid, insoluble in $H_2O$ soluble in polar and protic organic solvents | A |
| R-G-COONa | 8 | $R-[CO-(CH_2)_3COONa]_5$ | $C_{52}H_{55}O_{31}Na_5$ | 1.310 | - | 46,50 | 45,7 | powdery yellow-brown solid, highly melting with decomposition, sol. in $H_2O$ insol. in common organic solvents | A |
| D-G-COONa | 9 | $D-[CO-(CH_2)_3-COONa]_6$ | $C_{56}H_{63}O_{33}Na_6$ | 1.425 | - | 42,70 | 42,0 | powdery yellow-brown solid, highly melting with decomposition, sol. in $H_2O$ insol. in common organic solvents. | A |

TABLE 1 (follows)

| Compound | No. | Theoretical formula | Raw formula | $\overline{PM}$ calc. (dalton) | $\overline{PM}$ found[a] (dalton) | Q% (U.V.) calc. | Q% (U.V.) found[b] | ASPECT AND SOLUBILITY | Synthesis reaction |
|---|---|---|---|---|---|---|---|---|---|
| D-S-COONa | 10 | $D-(CO-CH_2-CH_2-COONa)_5$ | $C_{52}H_{51}O_{33}Na_6$ | 1.341 | - | 45,38 | 46,1 | Powdery yellow-brown solid, highly melting with decomposition, soluble in $H_2O$ insol. in common organic solvents. | A |
| R-S-PEG (via 5) | 11 | $R-[CO-(CH_2)_3-CO-O-PEG-OMe]_5$ | $C_{127}H_{210}O_{66}$ | 2.790 | 2.756 | 21,88 | 22,3 | red-brown oil highly viscous at room temperature; sol. in $H_2O$ and in the common halogenated and protic solvents, pratically insoluble in ether and gasoline | C |
| P-S-PEG (via 1) | 11' | $R-[CO-(CH_2)_3-CO-O-PEG-OMe]_5$ | $C_{127}H_{210}O_{66}$ | 2.790 | 2.805 | 21,88 | 21,9 | see 11 | D |
| P-S-PEG (via 2) | 11" | $R-[CO-(CH_2)_3-CO-O-PEG-OMe]_5$ | $C_{127}H_{210}O_{66}$ | 2.790 | 2.711 | 21,88 | 20,5 | see 11 | E |
| R-S-PEG (via 3) | 12 | $R-(CO-CH_2-CH_2-CO-O-PEG-OMe)_5$ | $C_{122}H_{200}O_{66}$ | 2.720 | 2.815 | 22,44 | 21,3 | red-brown oil viscous; sol. in $H_2O$ and common halogenated and protic solvents, pratically insoluble in ether and gasoline | D |

TABLE 1 (follows)

| Compound | No. | Theoretical formula | Raw formula | $\overline{PM}$ calc. | $\overline{PM}$ found[a] (dalton) | Q% (U.V.) calc. | Q% (U.V.) found[b] | ASPECT AND SOLUBILITY | Synthesis reaction |
|---|---|---|---|---|---|---|---|---|---|
| D-S-PEG (via 3) | 13 | $D-[CO-(CH_2)_3-CO-O-PEG-OMe]_6$ | $C_{148}H_{249}O_{75}$ | 3.225 | 3.351 | 18,86 | 19,6 | red-brown oil highly viscous at room temperature; sol. in $H_2O$ and in the common polar halogenated and protic solvents, pratically insoluble in ether and gasoline | E |
| D-S-PEG (via 2) | 14 | $D-(CO-CH_2-CH_2-CO-O-PEG-OMe)_6$ | $C_{142}H_{237}O_{75}$ | 3.141 | 3.266 | 19,37 | 20,7 | red-brown oil with chemical-physical properties very similar to 13 | C |

Note

a) values (± 3%) obtained with osmometer Wescan Instruments-Mod.233 using as ebullioscopic solvent acetone at the temperature of 35%

b) the values (± 1%) reported in the table are the the average of at least three independent measurements, calibrating curves recorded on R and D pure and dried, both in 0.1 N NaOH and in 95° EtOH; $\lambda_{anal.}$ at the adsorption peaks in UV (250-270 nm) and visible (340)370 nm) range.

c) with MeO-PEG-OH there is means the average statistical formula Me $O-(CH_2CH_2O)_7H$.

The compounds of the present invention have been the subject of a pharmacological tests aiming from one side to asses the toxicity and on the other side the activity; in this connection the activity has been particularly determined in comparison with the starting bioflavonoids (rutin and diosmin) using consequently the related specific tests.

For the toxicity , $LD_{50}$ has been calculated at equimolar doses , namely taking it into account that to 1000 mg of rutin correspond about 3000 mg of sodium salt and aboute 5000 mg of ester.

TABLE 2

| Compound | No. | $LD_{50}$ | Diffusion: test (1) Evans blue test-rat | Histamine induced cedema(2) |
|---|---|---|---|---|
| R-G-COONa | 8 | >3000 mg/kg | activity like that of rutin at equimolar doses | activity higher than that of rutin and equimolar doses |
| D-G-COONa | 9 | " | " | " |
| D-S-COONa | 10 | " | " | " |
| R-G-PEG (via 5) | 11 | >5000 mg/kg | " | " |
| R-G-PEG (via 1) | 11' | " | " | " |
| R-G-PEG (via 2° | 11" | " | " | " |
| R-S-PEG (via 3) | 12 | " | " | " |
| D-G-PEG (via 2) | 13 | " | " | " |
| D-S-PEG (via 7) | 14 | " | " | " |

1) Evans Bleu diffusion by jaluronidase.

Method

The test has been carried out on four groups of Sprague-Dawley rats weighing 200-250 g.

One hour after the administration the animals have been subjected to anaestesy with ethyl urethane (1.25 g/ kg i.p.), and then the ventral area has been depilated in order to permit the inoculation, in two predetermined symmetrical points, of 0.1 ml of 1% Evans Bleu in which 75 U.I. of jaluronidase were present.

One hour after the inoculation, the determination of the diffusion area of the colouring compound as induced by the jaluronidase has been carried out , the contour of the coloured areas being reported on transparent paper to permit their quantitative calculation from their area.

Results

One hour from the inoculation, the values of the diffusion areas expressed in square millimeters, have been found reduced in the pre-treated animals, particularly in those pre-treated with the tested compound , with respect to those of the control animals.

2) Oedema induced from histamine in the rat

Method

The test has been carried out to evaluate the capacity of tested compound of reducing the oedema induced by administration in the plantar surface of the rat of a solution (1mg/ml) of hystamine, which, as it is well known , is active on the capillary endotelium more permeable to the water and to the plasmatic proteins.

For the test four groups of Sprague-Dawley rats weaking 200-250 g have been used.

30 minutes after the administration the histamine solution has been inoculated in a volume of 0.1 ml per rat.

The variation of the leg volume has been evaluated by means of pletismometer.

For each group of animals the initial volume of the leg has been assessed (V 0); then the administration of the tested compound has been carried out as above indicated. One hour after the inoculation of the histamine the volume of the leg has been assessed again (V 1- V 0= Δ V). 3) Inflammation of the rabbit eye.

The protection induced by the pretreatment for 14 days with some compounds of the invention has been evaluated , for a dose corresponding to 100 mg/kg of the starting bioflavonoid (rutin or diosmine), with respect to the inflammatory reaction as induced in the rabbit eye by instilling a solution 0.1 N of NaOH.

The evaluation has been carried out by determining in the aqueous humour the content of plasmatic proteins and the number of leucocites. In the tables 3 and 4 the experimental results are reported.

TABLE 3

| Concentration of proteins (mg/ml) in the aqueous humour (average values) | | | | |
|---|---|---|---|---|
| | TREATED ANIMAL | | CONTROL ANIMAL | |
| | right eye | left eye | right eye | left eye |
| R-G-COONa | 3.95 | 2.84 | 6.42 | 3.08 |
| D-G-COONa | 4.3 | 3.0 | 6.3 | 2.95 |
| R-G-PEG * | 4.27 | 2.93 | 6.6 | 3.15 |
| D-G-PEG * | 4.34 | 2.88 | 6.45 | 2.87 |

* where PEG is a carrier as defined on page 5 (8)

TABLE 4

| Number of leucocites in 1 ul of aqueous humour (average values) | | | | |
|---|---|---|---|---|
| | TREATED ANIMAL | | CONTROL ANIMAL | |
| | right eye | left eye | right eye | left eye |
| R-G-COONa | 2995 | 1648 | 6139 | 1683 |
| D-G-COONa | 3248 | 1635 | 6115 | 1615 |
| R-G-PEG * | 3224 | 1629 | 6093 | 1712 |
| D-G-PEG * | 3476 | 1689 | 6045 | 1754 |
| 4) Phlebotrophic activity | | | | |

* where PEG is a carrier as defined on page 5 (8)

The compounds R-G-COONa, D-G-COONa, R-G-PEG, D-G-PEG have been formulated as gel containing an amount of active principle corresponding to 4% of starting bioflavonoid and in form of capsules containing an amount of active ingredient corresponding to 200 mg of starting bioflavonoid.

The test has been carried out on four group of ten voluntary patients aged between 47 and 76 years , affected from prevaricosis states and varicosis states. The symptoms were evaluated according to the following scale:
Absent
slight
medium
heavy

The treatment has been carried out for 3 weeks or 6 weeks by administering two capsules per day and topically applying in the gel three times per day.

All the symptoms were classified as medium or heavy.

At the end of the treatment all the symptoms were classified as "absent" or "slight"

5) Gynaecological antiflogistic activity

6 voluntary femole patients (aged between 22 and 58 years ) affected by flogistic and/or distrophic states of the genital organs have been treated for ten days with a lotion (150 ml) having the following composition.
R-G-PEG: 4 g
benzalkonium chloride g 0.025
perfume g 0.005
demineralized water enough to g 100

At the end of the test the examination showed the disappearance of the flogistic state.

Consequently the compounds of the present invention are active in the case of flogistic and distrophic states of the female genital organs; vulvo vaginites and exsocervicites ; intimate hygiene during the puepery and for the prophilaxis before and after surgical gynaecological intervention.

6) Activity on the microcirculation

Four voluntary patients (two males and two females aged between 66 and 79 years), suffering from cerebrovascular

insufficiency such as fertigo, mental deterioration, memory and concentration lackness; this is of peripheral microcirculation such as painfull spasm of the legs , cool sensation, have been treated for 60 days with two capsules per day containing the amount of active principle R-G-PEG corresponding to 200 mg of rutin.

The therapy, has demonstrated a reduction of the symptomatology of cerebrovascular insufficiency, the related functionality being improved.

At the same time the trophic state of the skin and of the legs has been improved, with disappearance of the painful spasms.

The pharmaceutical compositions according to the present invention contemplate formulation for oral use , ( capsules, tablets, solutions) for parenteral use and for topical use.

They are prepared according to the normal pharmaceutical techniques and with the use of the standard excipients and vehicles.

Both the unitary dosages and the posology correspond to the already known and used figures for the starting bioflavonoids.

## Claims

1. Derivatives of glycoside bioflavonoids having general formula

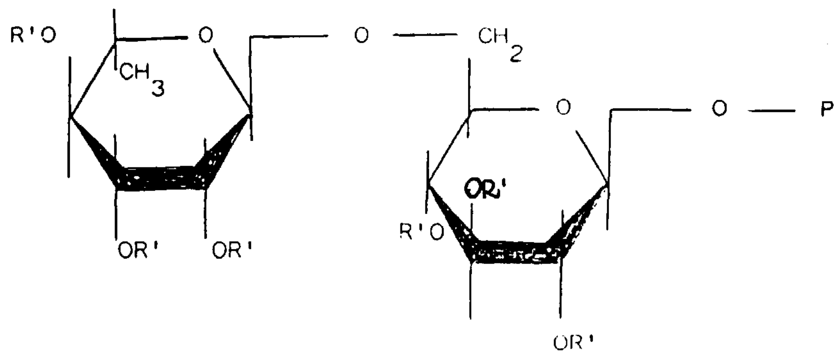

wherein P represents the flavonic residue containing phenolic hydroxyls and R' represents H, (CO-R-CO-O-X) , wherein X is selected among H, the cation of a pharmaceutically acceptable base and the radicals of polyoxyethylenglycols, and polyoxypropylenglycols and their O-monomethylethers, and R is an alkyl or aryl radical of dicarboxylic acid, provided that at least one R' substituent is different from H.

2. Derivatives according to claim 1, characterized in that said glycoside bioflavonoids are selected among rutin and diosmin.

3. Derivatives according to claim 1, characterized in that R is the radical of succinic or glutaric acid.

4. Derivatives according to claim 1, characterized in that X is an alkali metal.

5. Derivatives according to claim 4, characterized in that said alkaly metal is sodium.

6. Derivatives according to claim 1, characterized in that X is polyoxyethyleneglycol 350-0-methylether.

7. A process for the preparation of the derivatives of claim 1, characterized by the steps of:

   a) reaction of the glycoside with the anhydride of the aliphatic or aromatic dicarboxylic acid, in the presence of an organic base, under controlled conditions of temperature in the reaction time, taking care that the reaction environment is anhydrous and whatever photochemical action on the starting glycoside is prevented.
   b) reaction of the free carboxyls of the resulting ester-carboxyl derivative with a reactant selected among pharmaceuticall acceptable base, polyoxyethylenglycols, polyoxypropyleneglycols and their O-monomethylethers.

**8.** A process according to claim 7, characterized in that said dicarboxylic acid is succinic acid or glutaric acid and said starting glycoside is rutin or diosmin.

**9.** A process according to claim 7, characterized in that said step (a) is carried out at temperature no higher than 60°C.

**10.** A process according to claim 7, characterized in that said step (a) is carried out in an organic solvent carefully anhydrified.

**11.** A process according to claim 7, characterized in that said step (b) when said base is an inorganic base, the reaction is carried out in aqueous solution, said inorganic base being carbonate, bicarbonate, or hydrate of the desired cation.

**12.** A process according to claim 11, characterized in that said inorganic base is sodium bicarbonate , carbonate or hydrate.

**13.** A process according to claim 7, characterized in that said step (b), when said base is an organic base , the latter is directly added in form of solution in an organic solvent, to the ester carboxylic derivative obtained from the step (a).

**14.** A process according to claim 7, characterized in that in the case of esterification of the ester-carboxylic derivative obtained from the step (a) with a polyether, the reaction is carried out through the active amide of said ester-carboxylic derivative of the glycoside bioflavonoid and/or of the carboxlyic hemiester of polyoxyethylenglycols, polyoxypropylen glycols or their O-monomethylethers.

**15.** A process according to claim 14, characterized in that said active amides are selected among imidazolide and benzotriazolide of the carboxylic acid.

**16.** Pharmaceutical compositions , characterized by comprising as the active ingredient a water soluble derivative according any of the claims 1-6, together with the standard excipients and vehicles.

**17.** Pharmaceutical compositions according to claims 16, characterized in that said water soluble derivative is derivative of rutin and of diosmin.

**Patentansprüche**

**1.** Glykosid-Bioflavonoid-Derivate mit der allgemeinen Formel

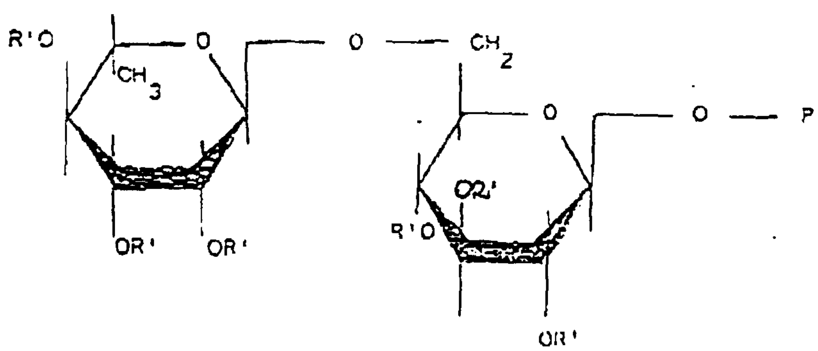

in der P den Flavonrest darstellt, der phenolische Hydroxylreste enthält und R' H, (CO-R-CO-O-X) darstellt, wobei X ausgewählt ist aus H, dem Kation einer pharmazeutisch verträglichen Base und den Resten Polyoxyethylenglykol und Polyoxypropylenglykol und deren O-Monomethylethern und R ein Alkyl-oder Arylrest oder eine Dicarbonsäure ist, mit der Maßgabe, daß mindestens ein R'-Substituent sich von H unterscheidet.

**2.** Derivate nach Anspruch 1,
**dadurch gekennzeichnet, daß**
die Glykosid-Bioflavonoide ausgewählt sind aus Rutin und Diosmin.

**3.** Derivate nach Anspruch 1,
**dadurch gekennzeichnet, daß**
R der Rest der Bernstein- oder Glutarsäure ist.

**4.** Derivate nach Anspruch 1,
**dadurch gekennzeichnet, daß**
X ein Alkalimetall ist.

**5.** Derivate nach Anspruch 4,
**dadurch gekennzeichnet, daß**
das Alkalimetall Natrium ist.

**6.** Derivate nach Anspruch 1,
**dadurch gekennzeichnet, daß**
X Polyoxyethylenglykol 350-0-methylether ist.

**7.** Verfahren zur Herstellung der Derivate nach Anspruch 1,
**gekennzeichnet durch**
die Schritte:

a) Reaktion des Glykosids mit dem Anhydrid der aliphatischen oder aromatischen Dicarbonsäure in Gegenwart einer organischen Base bei kontrollierten Temperaturbedingungen während der Reaktionszeit, wobei darauf geachtet wird, daß die Reaktion unter Abwesenheit von Wasser und unter Vermeidung jeglicher photochemiscber Einwirkung auf das Ausgangsglykosid durchgeführt wird,
b) Reaktion der freien Carboxylreste des entstehenden Ester-Carboxyl-Derivats mit einem Reaktanten, ausgewählt aus pharmazeutisch verträglichen Basen, Polyoxyethylenglykolen, Polyoxypropylenglykolen und deren O-Monomethylethern.

**8.** Verfahren nach Anspruch 7,
**dadurch gekennzeichnet, daß**
die Dicarbonsäure Bernstein- oder Glutarsäure und das Ausgangsglykosid Rutin oder Diosmin ist.

**9.** Verfahren nach Anspruch 7,
**dadurch gekennzeichnet, daß**
der schritt (a) bei einer Temperatur von nicht über 60°C ausgeführt wird.

**10.** Verfahren nach Anspruch 7,
**dadurch gekennzeichnet, daß**
der schritt (a) in einem organischen Lösungsmittel durchgeführt wird, das sorgfältig von Wasser befreit wurde.

**11.** Verfahren nach Anspruch 7,
**dadurch gekennzeichnet, daß**
in Schritt (b) die Reaktion in wässriger Lösung durchgeführt wird, wenn die Base eine anorganische Base ist, wobei die anorganiscne Base Carbonat, Bicarbonat oder das Hydrat des gewünschten Kations ist.

**12.** Verfahren nach Anspruch 11,
**dadurch gekennzeichnet, daß**
die anorganische Base Natriumbicarbonat, Carbonat oder Hydrat ist.

**13.** Verfahren nach Anspruch 7,
**dadurch gekennzeichnet, daß**
in Schritt (b), wenn die Base eine organische Base ist, letztere direkt als Lösung in einem organischen Lösungsmittel dem aus Schritt (a) erhaltenen Ester-Carboxyl-Derivat zugegeben wird.

**14.** Verfahren nach Anspruch 7,
**dadurch gekennzeichnet, daß**
im Falle der Veresterung des aus Schritt (a) erhaltenen Ester-Carboxyl-Derivats mit einem Polyether, die Reaktion über das aktive Amid des Ester-carboxyl-Derivats des Glykosid-Bioflavonoids und/oder des Carboxyl-Hemiesters

von Polyoxyethylenglykolen, Polyoxypropylenqlykolen oder deren O-Monomethylethern durchgeführt wird.

**15.** Verfahren nach Anspruch 14,
**dadurch gekennzeichnet, daß**
die aktiven Amide ausgewählt werden aus dem Imidazolid und dem Benzotriazolid der Carbonsaure.

**16.** Pharmazeutische zusammensetzungen,
**dadurch gekennzeichnet, daß**
sie als aktiven Bestandteil ein wasserlösliches Derivat nach einem der Ansprüche 1-6 zusammen mit standardisierten Trägern und Vehikeln umfaßt.

**17.** Pharmazeutische Zusammensetzungen nach Anspruch 16,
**dadurch gekennzeichnet, daß**
das wasserlösliche Derivat ein Derivat von Rutin oder Diosmin ist.

## Revendications

**1.** Dérivés de bioflavonoïdes ayant la formule général :

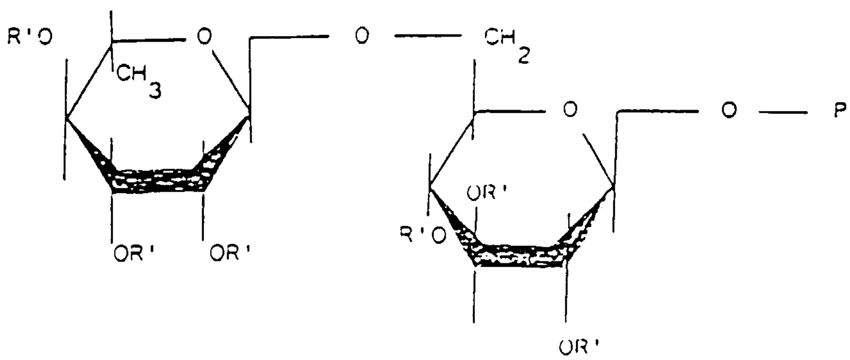

dans laquelle P représente le résidu flavonique contenant des hydroxyles phénoliques et R' représente H, (CO-R-CO-O-X), où X est sélectionné parmi H, le cation d'une base acceptable pharmaceutiquement et les radicaux de polyoxyéthylène glycols, et de polyoxypropylène glycols et leurs O-monométhyléthers et R est un radical alkyle ou aryle d'un acide bicarboxylique, étant donné qu'au moins un substituant R' est différent de H.

**2.** Dérivés selon la revendication 1, caractérisés en ce que lesdits bioflavonoïdes de glycoside sont sélectionnés parmi le rutine et la diosmine.

**3.** Dérivés selon la revendication 1, caractérisés en ce que R est le radical de l'acide succinique ou glutarine.

**4.** Dérivés selon la revendication 1, caractérisés en ce que X est un métal alcalin.

**5.** Dérivés selon la revendication 4, caractérisés en ce que ledit métal alcalin est le sodium.

**6.** Dérivés selon la revendication 1, caractérisés en ce que X est le polyoxyéthylène glycol 350-0-méthyléther.

**7.** Procédé pour la préparation des dérivés selon la revendication 1, caractérisé par les étapes de :

a) mise en réaction du glycoside avec l'anhydride de l'acide bicarboxylique aliphatique ou aromatique, en la présence d'une base organique, dans des conditions contrôlées de température pendant la durée de réaction, en prenant soin que l'environnement de réaction soit anhydre et que toute action photochimique sur le glycoside de départ soit empêchée;
b) mise en réaction des carboxyles libres du dérivé ester-carboxyle résultant avec un réactif sélectionné parmi une base pharmaceutique acceptable, des polyoxyéthylène glycols, des polyoxypropylène glycols et leurs O-monométhyléthers.

**8.** Procédé selon la revendication 7, caractérisé en ce que ledit acide bicarboxylique est l'acide succinique ou l'acide glutarique et ledit glycoside de départ est la rutine ou la diosmine.

**9.** Procédé selon la revendication 7, caractérisé en ce que ladite étape (a) est exécutée à une température ne dépassant pas 60°C.

**10.** Procédé selon la revendication 7, caractérisé en ce que ladite étape (a) est exécutée dans un solvant organique anhydrifié avec soin.

**11.** Procédé selon la revendication 7, caractérisé en ce que lors de ladite étape (b), lorsque ladite base est une base non organique, la mise en réaction est exécutée dans une solution aqueuse, ladite base non organique étant du carbonate, du bicarbonate ou un hydrate du cation souhaité.

**12.** Procédé selon la revendication 11, caractérisé en ce que ladite base non organique est du bicarbonate, du carbonate ou un hydrate de sodium.

**13.** Procédé selon la revendication 7, caractérisé en ce que lors de ladite étape (b), lorsque ladite base est une base organique, cette dernière est ajoutée directement, sous la forme d'une solution dans un solvant organique, au dérivé carboxylique obtenu à l'étape (a).

**14.** Procédé selon la revendication 7, caractérisé en ce que, dans le cas de l'estérification du dérivé d'ester carboxylique obtenu à l'étape (a) avec un polyéther, la réaction est exécutée via l'amide actif dudit dérivé d'ester carboxylique du bioflavonoïde de glycoside et/ou de l'émyesther carboxylique, de polyoxyéthylène glycols, polyoxypropylène glycols ou leurs O-monométhyléthers.

**15.** Procédé selon la revendication 14, caractérisé en ce que lesdits amides actifs sont sélectionnés parmi l'imidazolide et le benzotriazolide de l'acide carboxylique.

**16.** Compositions pharmaceutiques, caractérisées par le fait de comprendre à titre d'ingrédient actif, un dérivé soluble dans l'eau selon l'une quelconque des revendications 1 à 6, conjointement avec les excipients et les véhicules standards.

**17.** Compositions pharmaceutiques, caractérisées en ce que ledit dérivé soluble dans l'eau est un dérivé de rutine et de diosmine.